(19) Europäisches Patentamt
European Patent Office
Office européen des brevets

(11) Publication number: **0 557 087 B1**

# (12) EUROPEAN PATENT SPECIFICATION

(45) Date of publication of patent specification: **06.12.95**

(51) Int. Cl.[6]: **A61K 7/06**, A61K 7/11

(21) Application number: **93301184.3**

(22) Date of filing: **18.02.93**

(54) **Aqueous aerosol hair-spray compositions with a relatively low content of volatile organic compounds.**

(30) Priority: **21.02.92 US 838987**

(43) Date of publication of application:
**25.08.93 Bulletin 93/34**

(45) Publication of the grant of the patent:
**06.12.95 Bulletin 95/49**

(84) Designated Contracting States:
**AT BE CH DE DK ES FR GB IE IT LI LU NL PT SE**

(56) References cited:
**EP-A- 0 205 306**
**GB-A- 2 132 653**
**GB-A- 2 197 352**
**US-A- 5 053 218**
**US-A- 5 164 177**

(73) Proprietor: **Revlon Consumer Products Corporation**
**625 Madison Avenue**
**New York, NY 10022 (US)**

(72) Inventor: **Goldberg, Marvin Eugene**
**4 Palomino Way**
**Marlboro,**
**New Jersey 07746 (US)**
Inventor: **Bhambhani, Malti Vishin**
**7 Greensview Drive**
**Scotch Plains,**
**New Jersey 07076 (US)**
Inventor: **Brandon, Arthur**
**112 Sierra Vista Lane**
**Valley Cottage,**
**New York 10989 (US)**

(74) Representative: **Sanderson, Laurence Andrew et al**
**SANDERSON & CO.**
**European Patent Attorneys**
**34, East Stockwell Street**
**Colchester**
**Essex CO1 1ST (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

The invention relates to aqueous aerosol hair-spray compositions with a relatively low content of volatile organic compounds.

Volatile organic compounds (commonly referred to as "VOCs") are widely used in aerosol spray products such as hair-sprays, deodorants and the like. Some environmentalists believe that VOCs have an adverse effect on the ozone layer of the earth's atmosphere. Environmental lobbies in various countries are therefore urging the enactment of legislation which will reduce or eliminate the use of VOCs in personal-care products marketed in the aerosol spray form. Legislation has already been enacted in some countries which limits the amount of VOCs in an aerosol product to 80%.

Amongst the most widely used aerosol spray products, so much so that they have become ubiquitous in all except the most backward parts of our society, are aerosol hair-sprays - which are made up basically from resins in conjunction with a propellant which enables the resin to be applied to the hair in a finely-dispersed spray, that quickly dries on the hair and there exhibits hair-holding power.

If however the proportion of VOCs in such hair-sprays is reduced, then the now-missing VOCs must be replaced by other constituents of a nonvolatile nature. There are however only relatively few nonvolatile liquids which would be suitable as replacements for VOCs. The obvious choice as a VOC-replacement, for economic and safety reasons, would be water - which as compared with VOCs counts as non-volatile, and of course is environmentally friendly.

There are however more problems in achieving hair-spray compositions with a relatively low content of VOCs than can be solved by the mere replacement of a proportion of the VOCs by water, thus by shifting over to low-VOC-content semi-aqueous systems. Any such change also involves significantly changing other formulation constituents. Thus for example, if the proportion of VOCs present in the traditional anhydrous systems is reduced and the shortfall is then replaced with the preferred non-VOC ingredient namely water, the result is an essentially aqueous system - but in that case the water-insoluble resins traditionally present in anhydrous hair-sprays must be replaced with water-soluble resins.

Attempts to reformulate hair-sprays as semi-aqueous systems containing water-soluble resins have however so far proved unsatisfactory. None has ever successfully incorporated more than 10% w/w of water, and none has proved commercially-acceptable, because they do not enjoy consumer approval. Experience till now indicates that semi-aqueous compositions cause hair to droop and become tacky under conditions of high humidity, and the presence of water seems to prolong the time needed for drying the hair. When semi-aqueous compositions containing water-soluble resins are sprayed, the water beads which accumulate on the hair are too large due to increased surface tension between the aqueous and nonaqueous phases. Furthermore, these semi-aqueous systems based on water-soluble resins dissolved in water also tend to be unsatisfactory in use with aerosol containers, since they often give rise to clogging of the spray nozzle or cause other problems to system malfunction.

We have sought to overcome the problems of semi-aqueous compositions and have now found it possible to develop low VOC-content hair-sprays, i.e. hair-sprays containing less than 80% of VOCs, which contain significantly more than the 10% w/w maximum water-content hitherto achieved yet nevertheless are able to provide an aesthetically-pleasing and commercially-acceptable product, provided they are reformulated as herein described.

According to this invention we now provide a low-VOC aerosol hair-spray composition, comprising propellant(s) together with a sprayable substrate having cosmetically-acceptable hair-spray resin(s) dissolved therein, in which the proportion of propellant(s) in the composition lies within the range of from 5 to 60% w/w of the whole composition, the balance being the substrate, and wherein the substrate apart from other herein specified constituents consists of or includes a mixture of water with one or more alcohol(s) the proportion of water present in the composition being from 15 to 40% w/w, the resin(s) dissolved in the substrate being water-soluble and present in a proportion of from 1.0% to 10.0% w/w of the Composition, and the substrate also includes volatile silicone(s) in a proportion of from 0.005 to 1.0% w/w of the composition and a neutralizer/plasticizer in a proportion of from 0.05% to 3.0% w/w of the composition.

As appears above, the proportions of ingredients in the compositions of this invention are stated there and elsewhere herein as percentages by weight, i.e. as % w/w.

The cosmetically-acceptable hair-spray resins as such are not really the concern of this invention, and they may for instance be any of those resins recognized as suitable for use in hair-sprays which resins are water-soluble. A variety of recognized water-soluble resins are suitable, including vinyl acetate/crotonic acid/vinyl neodecanoate copolymer, octyl acrylamide/acrylates/butyl amino ethyl methacrylate copolymer, vinyl acetate/crotonic acid, polyvinyl-pyrrolidone (PVP), polyvinyl pyrrolidone vinyl acetate copolymer, PVP acrylates copolymer, etc. Vinyl acetate/crotonic acid/vinyl neodecanoate copolymer is preferred.

The compositions of this invention must include propellant(s). The choice of the propellants for use in the compositions again is not really as such the concern of this invention, and the term "propellant(s)" is used to embrace any propellant compound or propellant mixture recognized as capable of effecting propellant action in aerosol systems. However, for general guidance it can be mentioned that suitable propellants include n-butane, isobutane, dimethyl ether, difluoroethane, chlorodifluoroethane, chlorodifluoromethane and other chlorofluorohydrocarbons, as well as mixtures of any of these.

The propellants which we currently prefer to employ are dimethyl ether, 1,1-difluoroethane, n-butane and isobutane, or mixtures thereof. It is in fact usually advantageous to employ a mixture of propellants, and above all a mixture of dimethyl ether with either n-butane and/or isobutane.

All these suggested propellants are manufactured by and commercially available from E. I. du Pont de Nemours, Wilmington, Delaware, United States of America, by whom they are sold under the trade names:

- dimethyl ether ........... Dymel A
- 1,1 difluoroethane ....... Dymel 152 A
- n-butane ................. Hydrocarbon A 17
- isobutane ................ Hydrocarbon A 31

but similar propellants are also commercially-available from a variety of other suppliers.

The amount of propellant used in the composition is preferably in the range of from 10 to 40% w/w of the whole composition.

The substrate must consist essentially of a water and alcohol(s) mixture such that the water forms from 15% to 40% w/w of the whole composition, and preferably so proportioned between the water and alcohol-(s) that the mixture forms from 40% to 70% w/w of the whole composition.

Suitable alcohols for use in the hair-spray compositions of this invention are $C_{2-6}$ organic alcohols, including ethanol, isopropanol and others. The preferred alcohol is ethanol, including denatured ethanol.

The water/alcohol mixture should generally comprise from 4 to 6 parts of water together with from 6 to 4 parts of alcohol, and thus from 40% to 60% w/w of water in the water and alcohol mixture.

The aqueous hair-spray compositions of this invention besides the above-discussed ingredients must also contain from 0.005 to 1.0% of volatile silicone(s), which may be either linear or cyclic silicones so long as they display a measurable vapour pressure, and will therefore usually be either cyclic or linear polydimethyl-siloxanes.

When linear polydimethylsiloxanes are employed it is preferred to use those having from 3 to 9 silicone atoms and conforming to the general formula:

$$(CH_3)_3Si\text{-}O\text{-}[Si(CH_3)_2\text{-}O]_n\text{-}Si(CH_3)_3 \qquad (I)$$

where n is an integer from 1 to 7 inclusive.

These linear volatile silicones generally have viscosities of less than 5 x $10^{-6}$ $m^2s^{-1}$ (or 5 centistokes) at 25°C.

We however find that it is usually more advantageous to employ cyclic volatile silicone(s), and in that case it is preferred to use those in which the number of silicon atoms in the cyclic silicone(s) is from 3 to 7, and especially those which conform to the general formula:

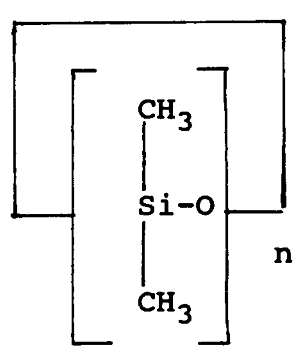

(II)

wherein n is an integer from 3 to 7 inclusive, above all those in which n is either 4 or 5.

These cyclic volatile silicones generally have viscosities of less than 1x$10^{-5}$ $m^2s^{-1}$ or 10 centistokes at 25°C.

The compositions of this invention must also contain from 0.05% to 3.0% w/w of what is here for convenience termed as"neutralizer/plasticizer". This term is used to indicate an ingredient which acts to make the resin less brittle. As such, plasticizers are known, and they act directly to make the resins less brittle. Also neutralizers as such are known, and they act directly to make the resins more water-soluble - but by doing so they indirectly thereby exert a plasticizing effect and make them less brittle.

Suitable plasticizers or neutralizers include methyl propanol, polyoxyethylene (20) sorbitan monooleate (known as Polysorbate 80), acetylated lanolin alcohol, cetyl acetate, propylene glucol, lauramide diethanolamine, dimethyl stearamine,the acetate of the polyethylene glycol ether(s) of lanolin alcohol with an average ethoxylation value of 10 (known as Laneth-10 acetate), the acetate of the polyethylene glycol ether(s) of lauryl alcohol with an average ethoxylation value of 10 (known as Laureth-10 acetate) and the polypropylene glycol-20 methyl glucose ether.

Within the parameters of this invention it is fully possible to formulate aerosol hair-spray compositions which due to the high loading of the sprayable substrate with water and despite the presence of alcohol(s) contain less than 80% of VOCs. In case it is possible within these parameters perversely to formulate the compositions so that they exceed the 50% limit let it be understood that the sum total of VOC-type propellant(s), alcohols(s) and volatile silicone(s) must not form more than 80% w/w of the whole composition.

When desired, the hair spray compositions may advantageously incorporate other ingredients, such as moisturizers, fragrances and surfactants.

If one or more moisturizers is/are incorporated, a proportion of from 0.00001 to 3.0% w/w thereof will usually serve to provide a satisfactory moisturizing effect upon the hair. Suitable moisturizers include hydrolyzed silk protein, panthenol, hydrolyzed wheat protein, etc.

If fragrance is incorporated into the composition, a proportion of from 0.05 to 1.0% w/w is likely to prove suitable.

The compositions according to the invention will preferably contain not only all the constituents mentioned above, but also in addition from 0.001 to 1.0% w/w of one or more surfactant(s), because surfactants reduce the surface tension between the aqueous and resin phases, and make it possible to create mist-type sprays containing a smaller droplet size.

Suitable surfactants include anionic, cationic, nonionic and amphoteric surfactants, preferably those having an hydrophilic/lipophilic balance (HLB) in the range of from 6 to 12, for instance the polyoxypropylene, polyoxyethylene ether of butyl alcohol that conforms to the formula:

$$C_4H_9(O\underset{\underset{CH_3}{|}}{C}HCH_2)_x(OCH_2CH_2)_yOH$$

where x has an average value of 28 and y has an average value of 35 (known as PPG 28 Buteth-35), dimethicone copolyol, the polymer of ethylene oxide that conforms to the general formula $H(OCH_2CH_2)_nOH$ where n has an average value of 25,the polyoxyethylene ether of lanolin having an average of 75 ethylene oxide units (known as PEG 75 lanolin),perfluoropolymethyl isopropyl ether,polysiloxane polyether copolymers, octoxynol-9, the polyexyethylene ether of hydrogenated castor oil having an average of 25 ethylene oxide units, poly- ethylene terephthalate, polyethylene glycol 25 glyceryl trioleate, the phosphate ester of the polyethylene glycol ether(s) of oleyl alcohol with an average of 3 ethylene oxide units (known as Oleth-3 phosphate), the phosphate ester of the polyethylene glycol ether(s) of cetyl alcohol that conforms to the formula:

$CH_3(CH_2)_{14}CH_2(OCH_2CH_2)_nOH$

where n has an average value of 10 (known as PPG-5-Ceteth-10 phosphate), and polyoxyethylene glycol-20 methyl glucose ether.

While other desirable ingredients are not necessarily excluded, we believe that the necessary ingredients specified above with or without the further optional but preferred ingredients which have been specifically mentioned, will suffice for the purposses we envisage - and it can be regarded as advantageous if there are no others, and the composition consists of these ingredients alone.

This composition of the invention though based on water-soluble resins is capable of imparting excellent holding power to hair without giving rise to droop or tackiness under high humidity conditions;and

furthermore, it works very well with standard aerosol containers, and will not clog the nozzle or cause other undesirable malfunction.

The compositions of this invention may be used in a method for styling hair comprising spraying the hair with an effective amount of the hair-spray composition, either before or preferably after it has been styled. In this method the composition is applied in one or more short bursts or sprays from the aerosol container, and the hair dries almost instantly. If applied after styling, then the applied spray will serve to set the hair in the desired style.

The invention will now be described in more detail, though only by way of illustration, with reference to the following examples, wherein all the amounts are stated in percentages by weight, i.e. as % w/w:

As a matter of convenience, certain short names are used for some ingredients in the Examples, which are more accurately defined as follows:

- Alcohol SD-40B .......... Ethanol denatured with brucine
- Dimethicone ..............
- Cyclomethicone ...........
- PPG 28 Buteth-35 ......... The previously-defined polyoxyropylene, polyoxyethylene ether of butyl alcohol
- Hydrocarbon A 31 ........ Isobutane
- Dymel A ................. Dimethyl ether

Example I

Preparation of Aqueous Substrate

An aqueous hair-spray substrate was made up from the following ingredients:

| | |
|---|---|
| **Alcohol/Water Mixture** | |
| Ethanol | 60.0000 |
| Water | 34.5599 |
| **Water-Soluble Resin** | |
| Vinyl acetate/crotonic acid/ vinyl neodecanoate copolymer | 4.7000 |
| **Volatile Silicone** | |
| Cyclomethicone | 0.1000 |
| **Neutralizer/Plasticizer** | |
| Amino methyl propanol | 0.4400 |
| **Moisturizer** | |
| Hydrolyzed silk protein | 0.0001 |
| Fragrance | 0.2000 |
| | 100% |

The aqueous hair-spray substrate was made up from these ingredients by a cold process using the following procedure. The ethanol was charged into a stainless steel vessel, and then with propeller stirring the amino methyl propanol was added, followed by the vinyl acetate/crotonic acid polymer. Thereafter the hydrolyzed silk protein was slowly added while vortexing, followed by the fragrance, cyclomethicone and water.

Preparation of Hair-Spray

The aqueous substrate prepared as described above was then mixed with propellant(s) in the usual manner and in the following proportions:

| | | |
|---|---|---|
| Aqueous Substrate | ..................... | 70 % |
| Isobutane | (Hydrocarbon A31) ....... | 10 % |
| Dimethylether | (Dymel A) ............... | 20 % |
| | | ----- |
| | | 100 % |
| | | ===== |

Treating the alcohol, volatile silicone and both propellants as VOCs, the sum total of all the VOCs in the finished product is 72.07% w/w.

Examples II - V

Preparation of Aqueous Substrates

Aqueous extra hold hair-spray substrates were made up from the following ingredients:

| | Ex.II | Ex.III | Ex.IV | Ex.V |
|---|---|---|---|---|
| **Alcohol/Water Mixture** | | | | |
| Alcohol SD-40B | 70.0000 | 70.0000 | 70.0000 | 70.0000 |
| Water | 24.5599 | 24.5599 | 24.5599 | 24.5599 |
| **Water-Soluble Resin** | | | | |
| Vinyl acetate/ crotonic acid/ vinyl neodecanoate copolymer | 4.7000 | 4.7000 | 4.7000 | 4.7000 |
| **Volatile Silicone(s)** | | | | |
| Dimethicone copolyol | 0.2000 | 0.2000 | 0.2000 | --- |
| Dimethicone | 0.1000 | --- | --- | 0.1000 |
| Cyclomethicone | --- | --- | 0.1000 | --- |
| **Neutralizer/ Plasticizer** | | | | |
| Amino methyl propanol | 0.4400 | 0.4400 | 0.4400 | 0.4400 |
| **Moisturizer** | | | | |
| Hydrolyzed Silk Protein | 0.0001 | 0.0001 | 0.0001 | 0.0001 |
| **Other Optional Ingredients** | | | | |
| Fragrance | --- | --- | --- | 0.1000 |
| **Surfactant** | | | | |
| PPG 28 Buteth-35 | --- | 0.1000 | --- | 0.1000 |
| | ------- | ------- | ------- | ------- |
| | 100.0 % | 100.0 % | 100.0 % | 100.0 % |
| | ======= | ======= | ======= | ======= |

The respective aqueous hair-spray substrates were made up in essentially the same manner as that already described in Example I above.

Preparation of Hair-Sprays

The aqueous substrates prepared as described above were then each mixed with the propellant in the following proportions:

| | |
|---|---|
| Aqueous Substrate<br>Isobutane (Hydrocarbon A31)<br>Dimethylether (Dymel A) | 70 %<br>10 %<br>20 %<br>100 % |

Treating the alcohol, volatile silicones and both propellants as VOCs, the sum totals of all the VOCs in the finished products were as follows... Ex.II = 79.21% w/w, Ex.III = 79.14% w/w, Ex.IV = 79.21% w/w and Ex.V = 79.21% w/w.

Example VI

Preparation of Aqueous Substrate

An aqueous ultimate hold hair-spray substrate was made up from the following ingredients:

| | |
|---|---|
| Alcohol/Water Mixture | |
| Alcohol SD-40B<br>Water | 67.0499<br>25.0000 |
| Water-Soluble Resin | |
| Vinyl acetate/crotonic neodecanoate copolymer | 7.0000 |
| Volatile Silicone | |
| Cyclomethicone | 0.1000 |
| Neutralizer/Plasticizer | |
| Amino methyl propanol | 0.6500 |
| Moisturizer | |
| Hydrolyzed silk protein<br>Other Optional Ingredients<br>Fragrance | 0.0001<br>0.2000<br>0.2000<br>100.0 % |

The aqueous hair-spray substrate was made up in essentially the same manner as that described in the previous Examples!

Preparation of Hair-Spray

The aqueous substrate prepared as described above was then mixed with the propellant in the following proportions:

| | | |
|---|---|---|
| Aqueous Substrate | ........................ | 70 % |
| Isobutane | (Hydrocarbon A31) ......... | 10 % |
| Dimethylether | (Dymel A) ................. | 20 % |
| | | ----- |
| | | 100 % |
| | | ===== |

Treating the alcohol, volatile silicone and both propellants as VOCs, the sum total of all the VOCs in the finished product was 77.005% w/w.

## Claims

1. A low-VOC aqueous aerosol hair-spray composition, comprising propellant(s) together with a sprayable substrate having cosmetically-acceptable hair-spray resin(s) dissolved therein, in which the proportion of propellant(s) in the composition lies within the range of from 5 to 60% w/w of the whole composition, the balance being substrate, and wherein the substrate apart from other herein specified constituents consists of or includes a mixture of water with one or more alcohol(s) the proportion of water present in the composition being from 15% to 40% w/w thereof, the resin(s) dissolved in the substrate being water-soluble and present in a proportion of from 1.0 to 10.0% w/w of the composition, and the substrate also includes volatile silicone(s) in a proportion of from 0.005 to 1.0% w/w of the composition and a neutralizer/ plasticizer in a proportion of from 0.05% to 3.0% w/w of the composition.

2. An aqueous aerosol composition as claimed in claim 1, wherein the propellant is one or a mixture of more than one of the following, namely: n-butane, isobutane, dimethyl ether, difluoroethane, chlorodifluoroethane, chlorodifluoromethane and/or propane.

3. An aerosol hair-spray composition as claimed in claim 1 or claim 2, wherein the propellant is one or a mixture of more than one of the following, namely: dimethyl ether, n-butane and/or isobutane.

4. An aerosol hair-spray composition as claimed in any of the preceding claims, in which the proportion of propellant(s) in the composition within the range of from 10% to 40% w/w of the whole composition.

5. An aqueous aerosol hair-spray composition as claimed in any of the preceding claims, wherein the resin is one or a mixture of more than one of the following water-soluble resins, namely: vinyl acetate/crotonic acid/vinyl neodecanoate copolymer, vinyl acetate/crotonic acid, polyvinyl pyrrolidone, polyvinyl pyrrolidone acrylates copolymer and polyvinyl pyrrolidone vinyl acetate copolymer.

6. An aqueous aerosol hair-spray composition as claimed in claim 5, wherein the water-soluble resin is vinyl acetate/crotonic acid/vinyl neodecanoate copolymer.

7. An aqueous aerosol hair-spray composition as claimed in any of the preceding claims, in which the alcohol(s) present are $C_2$-$C_6$ organic alcohols.

8. An aqueous aerosol hair-spray composition as claimed in claim 7, in which the alcohol is or includes ethanol.

9. An aqueous aerosol hair-spray composition as claimed in any of the preceding claims, wherein the water/alcohol mixture comprises water in a proportion of 40% to 60% w/w relative to said mixture.

10. An aqueous aerosol hair-spray composition as claimed in any of the preceding claims,, wherein the volatile silicone is or includes one or a mixture of more than one linear silicone(s) conforming to the general formula:

$$(CH_3)_3Si\text{-}O\text{-}[Si(CH_3)_2\text{-}O]_n\text{-}Si(CH_3)_3$$

where n is an integer from 1 to 7 inclusive.

11. An aqueous hair-spray composition as claimed in claim 10, wherein the volatile silicone is or includes one or a mixture of more than one linear silicone(s) having a viscosity of less than 5 x $10^{-6}\,m^2s^{-1}$.

12. An aqueous aerosol hair-spray composition as claimed in any of the preceding claims, wherein the volatile silicone is or includes one or a mixture of more than one cyclic silicone(s) conforming to the general formula.

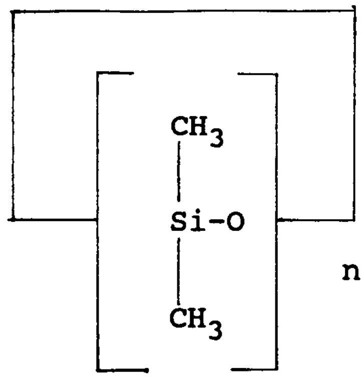

wherein n is an integer from 3 to 7 inclusive.

**13.** An aqueous aerosol hair-spray composition as claimed in claim 12, wherein the volatile silicone is or includes one or more cyclic silicone(s) in which n is either 4 or 5.

**14.** An aqueous aerosol hair-spray composition as claimed in claim 12 or claim 13, wherein the volatile silicone is or includes one or a mixture of more than one cyclic silicone(s) having a viscosity of less than 10 x $10^{-6} m^2 s^{-1}$.

**15.** An aqueous aerosol hair-spray composition as claimed in any of claims 12 to 14, wherein the volatile silicone is or includes cyclomethicone and/or dimethicone copolyol.

**16.** An aerosol hair-spray composition as claimed in any of the preceding claims, wherein the plasticizer/neutralizer is one or more of the following, namely: amino methyl propanol, polyoxyethylene (20) sorbitan monooleate, acetylated lanolin alcohol, cetyl acetate, propylene glycol, the acetate of the polyethylene glycol ether(s) of lanolin alcohol with an average ethoxylation value of 10, lauramide diethanolamine, dimethyl stearamine, the acetate of the polyethylene glucol ether(s) of lauryl alcohol with an average ethoxylation value of 10, and/or the polypropyleneglycol-20 methyl glucose ether.

**17.** An aqueous aerosol hair-spray composition as claimed in any of the preceding claims, which additionally contains from 0.001 to 1.0% of surfactant.

**18.** An aqueous aerosol hair-spray composition as claimed in claim 17, wherein the surfactant is an anionic, cationic, nonionic or amphoteric surfactant having an hydrophilic/lipophilic balance in the range of from 6 to 12.

**19.** An aerosol hair-spray composition as claimed in any of the preceding claims, which additionally contains from 0.00001 to 3% of moisturizer(s).

**20.** An aerosol hair-spray composition as claimed in claim 19, wherein the moisturizer is or includes hydrolyzed silk protein.

**21.** An aqueous aerosol hair-spray composition as claimed in any of the preceding claims, which additionally contains from 0.05% to 5.0% w/w of fragrance.

## Patentansprüche

**1.** Wäßrige Aerosol-Haarsprayzusammensetzung mit niedrigem Anteil an flüchtigen organischen Verbindungen (FOVs), umfassend ein oder mehrere Treibmittel zusammen mit einem versprühbaren Substrat, das kosmetisch akzeptable(s) Haarspray-Harz(e) darin gelöst hat, worin der Anteil des (der) Treibmittel in der Zusammensetzung im Bereich von 5 bis 60% Gew./Gew. der gesamten Zusammensetzung liegt, wobei der Rest Substrat ist und worin das Substrat abgesehen von anderen hierin angegebenen Bestandteilen aus einer Mischung aus Wasser mit einem oder mehreren Alkohol(en) besteht oder diese einschließt, wobei der wasseranteil, der in der Zusammensetzung anwesend ist, von 15% bis 40%

Gew./Gew. davon beträgt, das (die) in dem Substrat gelöste(n) Harz(e) wasserlöslich und in einem Anteil von 1,0 bis 10,0% Gew./Gew. der Zusammensetzung anwesend ist (sind), und das Substrat auch flüchtige(s) Silicon(e) in einem Anteil von 0,005 bis 1,0% Gew./Gew. der Zusammensetzung und ein Neutralisierungsmittel/Weichmacher in einem Anteil von 0,05% bis 3,0% Gew./Gew. der Zusammensetzung einschließt.

**2.** Wäßrige Aerosol-Zusammensetzung gemäß Anspruch 1, worin das Treibmittel eine oder eine Mischung von mehr als einer der folgenden Verbindungen ist, nämlich: n-Butan, Isobutan, Dimethylether, Difluorethan, Chlordifluorethan, Chlordifluormethan und/oder Propan.

**3.** Aerosol-Haarsprayzusammensetzung gemäß Anspruch 1 oder Anspruch 2, worin das Treibmittel eine oder eine Mischung von mehr als einer der folgenden Verbindungen ist, nämlich: Dimethylether, n-Butan und/oder Isobutan.

**4.** Aerosol-Haarsprayzusammensetzung gemäß einem der vorhergehenden Ansprüche, worin der Anteil des (der) Treibmittel in der Zusammensetzung im Bereich von 10% bis 40% Gew./Gew. der gesamten Zusammensetzung ist.

**5.** Wäßrige Aerosol-Haarsprayzusammensetzung gemäß einem der vorhergehenden Ansprüche, worin das Harz eines oder eine Mischung von mehr als einem der folgenden wasserlöslichen Harze ist, nämlich: Vinylacetat/Crotonsäure/Vinylneodecanoatcopolymer, Vinylacetat/Crotonsäure, Polyvinylpyrrolidon, Polyvinylpyrrolidonacrylatecopolymer und Polyvinylpyrrolidonvinylacetatcopolymer.

**6.** Wäßrige Aerosol-Haarsprayzusammensetzung gemäß Anspruch 5, worin das wasserlösliche Harz Vinylacetat/Crotonsäure/ Vinylneodecanoatcopolymer ist.

**7.** Wäßrige Aerosol-Haarsprayzusammenstzung gemäß einem der vorhergehenden Ansprüche, in welcher der (die) anwesende(n) Alkohol(e) $C_2$-$C_6$ organische Alkohole sind.

**8.** Wäßrige Aerosol-Haarsprayzusammensetzung gemäß Anspruch 7, in welcher der Alkohol Ethanol ist oder einschließt.

**9.** Wäßrige Aerosol-Haarsprayzusammensetzung gemäß einem der vorhergehenden Ansprüche, worin die Wasser/Alkoholmischung Wasser in einem Anteil von 40% bis 60% Gew./Gew. relativ zu der Mischung umfaßt.

**10.** Wäßrige Aerosol-Haarsprayzusammensetzung gemäß einem der vorhergehenden Ansprüche, worin das flüchtige Silicon ein oder eine Mischung von mehr als einem linearen Silicon ist oder einschließt, entsprechend der allgemeinen Formel:

$(CH_3)_3Si\text{-}O\text{-}[Si(CH_3)_2\text{-}O]_n\text{-}Si(CH_3)_3$,

worin n eine ganze Zahl von 1 bis einschließlich 7 ist.

**11.** Wäßrige Haarsprayzusammensetzung gemäß Anspruch 10, worin das flüchtige Silicon ein oder eine Mischung von mehr als einem linearen Silicon mit einer Viskosität von weniger als 5 x $10^{-6} m^2s^{-1}$ ist oder einschließt.

**12.** Wäßrige Aerosol-Haarsprayzusammensetzung gemäß einem der vorhergehenden Ansprüche, worin das flüchtige Silicon ein oder eine Mischung von mehr als einem cyclischen Silicon ist oder einschließt, entsprechend der allgemeinen Formel:

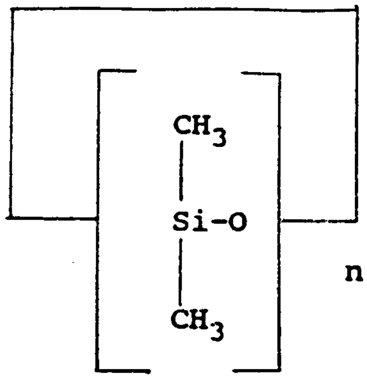

worin n eine ganze Zahl von 3 bis einschließlich 7 ist.

**13.** Wäßrige Aerosol-Haarsprayzusammensetzung gemäß Anspruch 12, worin das flüchtige Silicon ein oder mehrere cyclische Silicon(e) ist oder einschließt, in dem (denen) n entweder 4 oder 5 ist.

**14.** Wäßrige Aerosol-Haarsprayzusammensetzung gemäß Anspruch 12 oder Anspruch 13, worin das flüchtige Silicon ein oder eine Mischung von mehr als einem cyclischen Silicon mit einer Viskosität von weniger als $10 \times 10^{-6} m^2s^{-1}$ ist oder einschließt.

**15.** Wäßrige Aerosol-Haarsprayzusammensetzung gemäß einem der Ansprüche 12 bis 14, worin das flüchtige Silicon Cyclomethicon und/oder Dimethiconcopolyol ist oder einschließt.

**16.** Aerosol-Haarsprayzusammensetzung gemäß einem der vorhergehenden Ansprüche, worin der Weichmacher/das Neutralisierungsmittel eine oder mehrere der folgenden Verbindungen ist, nämlich: Aminomethylpropanol, Polyoxyethylen-(20)-Sorbitanmonooleat, acetylierter Lanolinalkohol, Cetylacetat, Propylenglycol, das Acetat des (der) Polyethylenglycolether(s) von Lanolinalkohol mit einem durchschnittlichen Ethoxylierungswert von 10, Lauramiddiethanolamin, Dimethylstearamin, das Acetat des (der) Polyethylenglycolether(s) von Laurylalkohol mit einem durchschnittlichen Ethoxylierungswert von 10 und/oder der Polypropylenglycol-20-methylglucoseether.

**17.** Wäßrige Aerosol-Haarsprayzusammensetzung gemäß einem der vorhergehenden Ansprüche, die zusätzlich von 0,001 bis 1,0% eines oberflächenaktiven Mittels enthält.

**18.** Wäßrige Aerosol-Haarsprayzusammensetzung gemäß Anspruch 17, worin das oberflächenaktive Mittel ein anionisches, kationisches, nicht-ionisches oder amphoteres oberflächenaktives Mittel mit einem hydrophilen/lipophilen Gleichgewicht im Bereich von 6 bis 12 ist.

**19.** Aerosol-Haarsprayzusammensetzung gemäß einem der vorhergehenden Ansprüche, die zusätzlich von 0,00001 bis 3% eines oder mehrerer Feuchthaltemittel enthält.

**20.** Aerosol-Haarsprayzusammensetzung gemäß Anspruch 19, worin das Feuchthaltemittel hydrolysiertes Seidenprotein ist oder einschließt.

**21.** Wäßrige Aerosol-Haarsprayzusammensetzung gemäß einem der vorhergehenden Ansprüche, die zusätzlich von 0,05% bis 5,0% Gew./Gew. Duftstoff enthält.

## Revendications

**1.** Composition aqueuse de laque pour cheveux sous forme d'aérosol à faible teneur en composés organiques volatiles (VOCs) comprenant un (des) agent(s) propulseur(s) associé(s) à un substrat vaporisable ayant une (des) résine(s) de laque pour cheveux cosmétiquement acceptable(s) dissoute-(s), dans laquelle la proportion de (des) agent(s) propulseur(s) dans la composition est dans le domaine de 5% à 60% en poids de la composition totale, le complément étant le substrat, et dans laquelle le substrat à part des autres constituants ici spécifiés, consiste en ou comprend un mélange d'eau avec

un ou plusieurs alcool(s), la proportion d'eau présente dans la composition étant de 15% à 40% en poids, la (les) résine(s) dissoute(s) dans le substrat étant soluble(s) dans l'eau et présente(s) en proportion de 1.0% à 10% en poids de composition, et le substrat comprend également une (des) silicone(s) volatile(s) en proportion de 0.005% à 1.0% en poids une composition, et un neutralisant plastifiant dans une proportion de 0.05% à 3.0% en poids de composition.

**2.** Composition aqueuse d'aérosol selon la revendication 1, dans laquelle l'agent propulseur est un, ou un mélange de plusieurs des composés suivants, c'est-à-dire: n-butane, isobutane, diméthyl éther, difluoroéthane, chlorodifluoroéthane, chlorodifluorométhane et/ou propane.

**3.** Composition de laque pour cheveux en aérosol selon la revendication 1 ou 2, dans laquelle l'agent propulseur est un, ou un mélange de plusieurs, des composés suivants, c'est-à-dire: diméthyl éther, n-butane et/ou isobutane.

**4.** Composition de laque pour cheveux en aérosol selon l'une quelconque des revendications précédentes, dans laquelle la proportion d'agent(s) propulseur(s) dans la composition est dans le domaine de 10% à 40% en poids de la composition totale.

**5.** Composition aqueuse de laque pour cheveux en aérosol selon l'une quelconque des revendications précédentes, dans laquelle la résine est une, ou un mélange de plusieurs des résines solubles dans l'eau suivantes, c'est-à-dire: copolymère acétate de vinyle/ acide crotonique/ néodécanoate de vinyle, acétate de vinyle/acide crotonique, polyvinyl pyrrolidone, copolymère de polyvinyle pyrrolidone acrylates et copolymère de polyvinyle pyrrolidone vinyle acétate.

**6.** Composition aqueuse de laque pour cheveux en aérosol selon la revendication 5, dans laquelle la résine soluble dans l'eau est le copolymère acétate de vinyle/acide crotonique/néodécanoate de vinyle.

**7.** Composition aqueuse de laque pour cheveux en aérosol selon l'une des revendications précédentes, dans laquelle l' (les) alcool(s) présent(s) est (sont) des alcools organiques en $C_2$-$C_6$.

**8.** Composition aqueuse de laque pour cheveux en aérosol selon la revendication 7, dans laquelle l'alcool est ou inclut l'éthanol.

**9.** Composition aqueuse de laque pour cheveux en aérosol selon l'une quelconque des revendications précédentes, dans laquelle le mélange eau/alcool comprend de l'eau en proportion de 40% à 60% en poids relativement audit mélange.

**10.** Composition aqueuse de laque pour cheveux en aérosol selon l'une quelconque des revendications précédentes, dans laquelle la silicone volatile est ou inclut une, ou un mélange de plusieurs silicones linéaires conformément à la formule générale suivante:

$$(CH_3)_3Si\text{-}O\text{-}[Si(CH_3)_2\text{-}O]_n\text{-}Si(CH_3)_3$$

dans laquelle n est un nombre entier de 1 à 7.

**11.** Composition aqueuse de laque pour cheveux selon la revendication 10, dans laquelle la silicone volatile est ou inclut une, ou un mélange de plusieurs silicone(s) linéaire(s) ayant une viscosité inférieure à 5 x $10^{-6} m^2s^{-1}$.

**12.** Composition aqueuse de laque pour cheveux en aérosol selon l'une quelconque des revendications précédentes, dans laquelle la silicone volatile est ou inclut une, ou un mélange de plusieurs silicone(s) cyclique(s) conformément à la formule générale suivante:

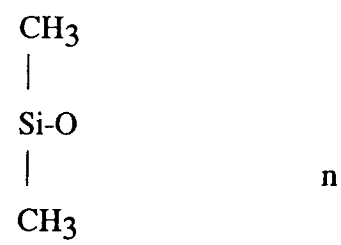

dans laquelle n est un nombre entier de 3 à 7.

13. Composition aqueuse de laque pour cheveux en aérosol selon la revendication 12, dans laquelle la silicone volatile est ou inclut une ou plusieurs silicone(s) cyclique(s) dans laquelle n est soit 4 ou 5.

14. Composition aqueuse de laque pour cheveux en aérosol selon la revendication 12 ou la revendication 13, dans laquelle la silicone volatile est ou inclut une ou un mélange de plus d'une silicone(s) cyclique-(s) ayant une viscosité inférieure à $10 \times 10^{-6} m^2s^{-1}$.

15. Composition aqueuse de laque pour cheveux en aérosol selon l'une quelconque des revendications 12 à 14, dans laquelle la silicone volatile est ou inclut la cyclométhicone et/ou le copolyol de diméthicone.

16. Composition de laque pour cheveux en aérosol selon l'une quelconque des revendications précédentes, dans laquelle le plastifiant neutralisant est un ou plusieurs des composés suivants, c'est-à-dire: amino méthyl propanol, polyoxyéthylène (20) sorbitane monooléate, lanoline alcool acétylé, acétate de cétyle, propylène glycol, l'acétate de (des) (l)'éther(s) de polyéthylène glycol de lanoline alcool avec une valeur d'éthoxylation moyenne de 10, lauramide diéthanolamine, diméthyl stéaramine, l'acétate de (des) (l')éther(s) polyéthylène glycol de lauryl alcool avec une valeur d'éthoxylation moyenne de 10, et/ou l'éther de polypropylène glycol-20 méthyl/glucose.

17. Composition aqueuse de laque pour cheveux en aérosol selon l'une quelconque des revendications précédentes, qui contient en plus de 0.001% à 1.0% d'agent tensioactif.

18. Composition aqueuse de laque pour cheveux en aérosol selon la revendication 17, dans laquelle l'agent tensioactif est anionique, cationique nonionique ou amphotérique ayant un rapport hydrophile/lipophile dans le domaine de 6 à 12.

19. Composition de laque pour cheveux en aérosol selon l'une quelconque des revendications précédentes, qui contient en plus de 0.00001% à 3% d'agent(s) humidifiant(s).

20. Composition de laque pour cheveux en aérosol selon la revendication 19, dans laquelle l'agent humidifiant est ou inclut la protéine de soie hydrolysée.

21. Composition aqueuse de laque pour cheveux en aérosol selon l'une quelconque des revendications précédentes, qui contient en plus de 0.05% à 5.0% en poids de parfum.